# EUROPÄISCHE PATENTSCHRIFT

(11) **EP 3 712 125 B1**
(45) Veröffentlichungstag und Bekanntmachung des Hinweises auf die Patenterteilung: **16.07.2025**
(21) Anmeldenummer: 19020230.9
(22) Anmeldetag: 22.03.2019
(51) Int. Cl.: C07C 7/09, C07C 11/02, C10G 3/00

(54) **VERFAHREN ZUM QUENCHEN EINES PRODUKTSTROMS**
METHOD FOR QUENCHING A PRODUCT FLOW
PROCÉDÉ DE REFROIDISSEMENT D'UN FLUX DE PRODUIT

(43) Veröffentlichungstag der Anmeldung: 23.09.2020
(73) Patentinhaber: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Ahlers, Bernd, 63128 Dietzenbach (DE); Drosdzol, Christopher, 60438 Frankfurt am Main (DE); Haag, Stéphane, 60598 Frankfurt am Main (DE); Williams, Bryce, 60437 Frankfurt am Main (DE); Castillo-Welter, Frank, 61381 Friedrichsdorf (DE)
(74) Vertreter: Air Liquide

(56) Entgegenhaltungen:
- WO-A1-93/12200
- CN-A- 108 358 739
- US-A- 3 674 890
- US-B1- 6 403 854

## Beschreibung

Die Erfindung betrifft ein Verfahren zum Quenchen eines Wasser und Kohlenwasserstoffe enthaltenden, fluiden Produktstroms aus einem Synthesereaktor.

Die Erfindung betrifft dabei ein Verfahren zum Quenchen spezifischer Produktströme, wie sie bei der Oxygenat-zu-Olefin-(OTO)-Synthese erhalten werden.

### Stand der Technik

Kurzkettige Olefine, insbesondere Propylen (Propen) und Ethylen (Ethen), gehören zu den wichtigsten Grundstoffen der chemischen Industrie. Dies liegt darin begründet, das ausgehend von diesen ungesättigten Verbindungen mit einer kurzen Kettenlänge Moleküle mit langkettigem Kohlenstoffgerüst und zusätzlichen Funktionalisierungen aufgebaut werden können.

Als Quelle für kurzkettige Olefine diente in der Vergangenheit vor allem das Steam-Cracking, d. h. die thermische Spaltung von Kohlenwasserstofffraktionen mit im wesentlichen gesättigten Kohlenwasserstoffen bei der Erdölverarbeitung. In den vergangenen Jahren wurden jedoch weitere Prozesse zur Herstellung von kurzkettigen Olefinen entwickelt. Dies ist zum einen durch steigende Nachfrage bedingt, die durch die vorhandenen Quellen nicht mehr gedeckt werden kann; zum anderen fordert die zunehmende Verknappung von fossilen Rohstoffen die Verwendung anderer Ausgangsstoffe.

Die sog. MTP- (Methanol-to-Propylen) oder auch MTO- (Methanol-to-Olefin) Verfahren zur Herstellung von Propylen und anderen kurzkettigen Olefinen gehen von Methanol als Ausgangsstoff aus. In diesen heterogen katalysierten Verfahren wird aus Methanol zunächst teilweise das Zwischenprodukt Dimethylether (DME) und aus einer Mischung von Methanol und Dimethylether dann nachfolgend im Olefinsynthesereaktor ein Produktgemisch aus Ethylen, Propylen und den isomeren Butenen als kurzkettige Olefine sowie schwereren Kohlenwasserstoffen mit mehr als vier C-Atomen gebildet. Zudem findet sich im Produktstrom Wasser, welches einerseits aus dem Prozessdampf stammt, der optional dem MTO-Reaktor als Verdünnungsmedium zugeführt wird, sowie aus dem im Synthesereaktor erzeugten Reaktionswasser.

Da außer Methanol und DME gegebenenfalls auch andere kurzkettige, Sauerstoff enthaltenden organischen Moleküle, beispielsweise andere Alkohole als Methanol, als Einsatzstoffe dienen können, wird auch verallgemeinernd von Oxygenat-zu-Olefin-Reaktionen (OTO-Reaktionen) bzw. Oxygenat-zu-Olefin-Synthesen (OTO-Synthesen) gesprochen.

Die sich anschließende Aufreinigung des Produktstroms des Synthesereaktors soll zum einen unerwünschte Nebenprodukte und nicht umgesetzte Edukte abtrennen und die einzelnen Kohlenwasserstofffraktionen möglichst rein herstellen. Üblicherweise wird hierzu im ersten Schritt ein Quenchsystem zur Anwendung gebracht, um eine schlagartige Abkühlung des Produktstroms des Synthesereaktors durch direkten Wärmetausch mit einem fluiden, zumeist flüssigen Quenchmedium, beispielsweise Wasser, zu bewirken. Ein erwünschter Nebeneffekt ist dabei eine gewisse Gaswaschwirkung des typischerweise zumindest teilweise als Gas oder Dampf vorliegenden Produktstroms.

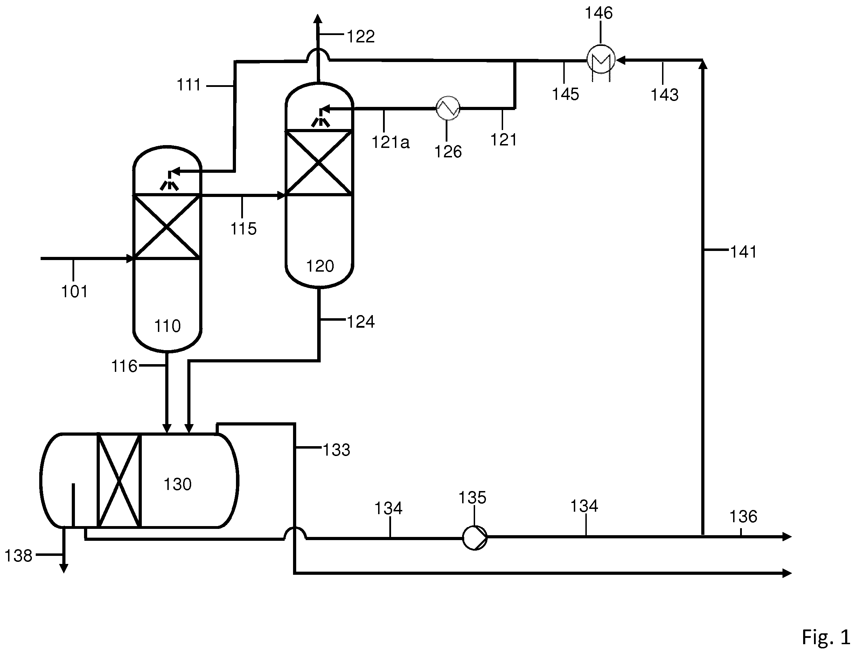

Fig. 1 zeigt schematisch ein in einer OTO-Anlage eingesetztes, konventionelles Quenchsystem. Über Leitung 101 wird der Produktstrom aus dem nicht dargestellten OTO-Synthesereaktor in eine erste Quenchstufe 110 geführt, in der Wasser teilweise kondensiert und ein Teil der schweren Bestandteile des Produktstroms wie Wasser und schwere Kohlenwasserstoffe vom gasförmigen OTO-Synthesereaktorprodukt getrennt werden. Optional kann der Produktstrom schon vor Eintritt in das Quenchsystem vorgekühlt werden. Der Produktstrom liegt bei Eintritt in die erste Quenchstufe hauptsächlich gasförmig oder dampfförmig vor, kann jedoch auch flüssige und/oder aerosolförmige Anteile beinhalten, insbesondere wenn eine Vorkühlung erfolgt. Nachfolgend wird daher von einem fluiden Produktstrom gesprochen. Insbesondere bei einem signifikanten Flüssiganteil im Produktstrom empfiehlt es sich, die Leitung 101 fallend zu verlegen.

In der ersten Quenchstufe 110 wird der Produktstrom mit dem über Leitung 111 eingebrachten, Wasser enthaltenden Quenchmedium in Kontakt gebracht.

Der größte Teil des im Produktstrom enthaltenen und mit dem Quenchmedium zugeführten Wassers wird in der ersten Quenchstufe 110 kondensiert und verlässt diese über Leitung 116 als erste Quenchfraktion.

Über Leitung 115 wird die verbleibende Gas- bzw. Dampfphase als erster Abflussstrom aus der ersten Quenchstufe 110 in die zweite Quenchstufe 120 geführt und dort über Leitung 121a mit einem Wasser enthaltenden zweiten Quenchmedium in Kontakt gebracht.

Über Leitung 124 wird die in der zweiten Quenchstufe 120 anfallende Flüssigkeit, die den größten Teil der im Quenchsystem kondensierten Kohlenwasserstoffe enthält, als zweite Quenchfraktion aus der zweiten Quenchstufe 120 ausgeleitet. Die verbleibende Gas- bzw. Dampfphase wird als zweiter Abflussstrom aus der zweiten Quenchstufe 120 über Leitung 122 ausgeleitet und der weiteren, nicht bildlich dargestellten Aufarbeitung zugeführt, die zumeist eine mehrstufige Destillation zur Gewinnung mehrerer Kohlenwasserstoffproduktfraktionen umfasst, darunter leichte Produktfraktionen wie Ethylen und Propylen.

Die in den beiden Quenchstufen erhaltenen Quenchfraktionen werden über Leitungen 116 und 124 in einen Phasenabscheider 130 zur Trennung von Wasser und Kohlenwasserstoffen eingebracht. In diesem Phasenabscheider 130 wird die vergleichsweise kalte, kohlenwasserstoffreiche zweite Quenchfraktion mit der relativ warmen, wasserreichen ersten Quenchfraktion vermischt. Dies führt zu einer teilweisen Verdampfung (Flashen) der in der zweiten Quenchfraktion gelösten, leichter flüchtigen Komponenten, vor allem niedrig siedende, kurzkettige Kohlenwasserstoffe, weshalb es sich empfiehlt, mittels Leitung 133 diese dampfförmige Fraktion vom Phasenabscheider 130 zum Kopfprodukt der zweiten Quenchstufe 120 in Leitung 122, also zum zweiten Abflussstrom zu überführen (Verbindung der Leitungen 122 und 133 nicht bildlich dargestellt).

Die in dem Phasenabscheider 130 abgetrennte, wässrige Phase wird in über Leitung 134 und Pumpe 135 abgeführt, teilweise über Leitungen 141, 143 und 145 sowie Wärmetauscher 146 als Quenchmedium zu den Quenchstufen zurückgeführt und diesen über die Leitungen 111 und 121 bzw. 121a aufgegeben. Dabei erfolgt in Wärmetauscher 146 eine Kühlung durch Wärmeintegration mit kalten Prozessströmen und in Wärmetauscher 126 eine Kühlung durch Kühlmedien, beispielsweise Kühlwasser. Überschüssiges Wasser wird über Leitung 136 aus dem Quenchsystem ausgeleitet und in nicht dargestellter Weise einer Methanol-Rückgewinnungskolonne zur Rückgewinnung von nicht umgesetzten Oxygenaten (DME, Methanol) und der Reinigungvorrichtung des Prozesswasserkreislaufs des OTO-Synthesereaktors zugeführt.

Die im Phasenabscheider 130 anfallende, kohlenwasserstoffreiche Phase enthält vor allem schwerere, leichter kondensierbare Kohlenwasserstoffe. Sie wird über Leitung 138 aus dem Phasenabscheider ausgeleitet und gemeinsam mit den über Leitungen 122 und 133 abgeführten, kohlenwasserstoffreichen Strömen einer in der Regel mehrstufigen Kohlenwasserstofffraktionierungsvorrichtung zugeführt. Nachteilig ist es dabei, dass die im Phasenabscheider 130 bereits erzielte Vortrennung der Kohlenwasserstoffe in einen leichter siedenden Anteil (abgeführt über Leitung 133) und einen schwerer siedenden Anteil (abgeführt über Leitung 138) nicht ausgenutzt wird. Alle innerhalb des Fließschemas der Fig. 1 anfallenden, kohlenwasserstoffreichen Ströme werden zu einem nicht dargestellten, stromabwärts angeordneten Dreiphasenabscheider geführt, dem sich üblicherweise eine mehrstufige destillative Fraktionierung der Kohlenwasserstoffe anschließt, so dass die im Phasenabscheider 130 bereits erzielte Trennung in leichtuns schwersiedende Kohlenwasserstofffraktionen nochmals mit zusätzlichem Energieaufwand wiederholt wird. Zusätzlich müssen mehr und größere Fraktionierungsstufen vorgesehen werden, als dies der Fall wäre, wenn bereits ein schwer siedender Kohlenwasserstoffanteil abgetrennt worden wäre.

Im Stand der Technik finden sich weitere Ausgestaltungen für mehrstufige Quenchsysteme auf dem Gebiet der Erfindung. So beschreibt Dokument US 7273961 B2 ein vierstufiges Quenchsystem, in dem mit Natronlauge der pH eingestellt werden soll. Daran schließt sich eine Methanol-Rückgewinnungskolonne an. Zudem ist dieses System für die Abscheidung von Katalysatorfeingut im unteren Bereich der ersten Quenchstufe und in einem zusätzlichen Abscheidebehälter ausgelegt. Im Abscheidebehälter sind drei Ströme getrennt, ein Wasserstrom mit Katalysatorfeinheiten, ein Wasserstrom mit einigen Alkoholen und ein Kohlenwasserstoffstrom.

Die erste Quenchstufe der US 7273961 B2 nutzt einen Teil des im gemeinsamen Quenchwasserkreislauf der zweiten und dritten Quenchstufe gekühlten Quenchwassers. Dieses Quenchmedium wird nicht in den Quenchwasserkreislauf der der zweiten und dritten Quenchstufe zurückgeführt. Daher muss der Quenchwasserdurchfluss der ersten Quenchstufe im Vergleich zum Quenchwasserkreislauf der zweiten und dritten Quenchstufe klein sein. Wie üblich sinkt die Temperatur des Quenchmittels über jede weitere Stufe, so dass das der dritten Quenchstufe zugeführte Quenchwasser auf eine niedrigere Temperatur als das der zweiten Quenchstufe zugeführte Quenchwasser abgekühlt wird.

Die vierte Quenchstufe der US 7273961 B2 verwendet einen separaten Quenchwasserkreislauf, der mit sauerstofffreiem Quenchwasser betrieben wird (z. B. der Bodenstrom der Methanol-Rückgewinnungskolonne). Ein Phasenabscheider kommt nicht zur Anwendung.

Problematisch ist es dabei, dass die Löslichkeit von leichten Kohlenwasserstoffen in heißem Quenchwasser wesentlich höher als in kälterem Quenchmedium ist. Über das Abkühlen im Quenchwasserkreislauf kommt es daher zur Abtrennung einer Kohlenwasserstoffphase, so dass für den Zwei-Phasen-Betrieb ausgelegte Komponenten, insbesondere Pumpen, verwendet werden müssen. Ferner ist nachteilig, dass Kohlenwasserstoffe, die in die Methanol-Rückgewinnungskolonne gelangen, dort zu Ablagerungen und Verstopfungen führen können.

Die Druckschrift US 8083951 B2 beschreibt ein zweistufiges Quenchsystem kombiniert mit einer Methanol-Rückgewinnungskolonne und getrennten Quenchwasserkreisläufen für die beiden Quenchstufen sowie eine Trennung von Katalysatorfeinteilen in beiden Quenchkreisläufen mittels "Mini-Hydrozyklonen". Eine Abtrennung der in den Quenchstufen kondensierten Kohlenwasserstoffe wird in dem Dokument nicht erwähnt. Die Nachteile des Systems sind daher im Wesentlichen dieselben wie schon für die US 7273961 B2 diskutiert.

Die Druckschrift CN108358739A betrifft eine Technologie zur Öl-Wasser-Gas-Trennung für ein Produkt aus einem Methanol-Propylen-Verfahren, insbesondere ein System und Verfahren zur Öl-Wasser-Gas-Trennung für für ein Methanol-Propylen-Verfahren. Offenbart wird ein Öl-Wasser-Gas-Trennsystem für ein Methanol-zu-Propylen-Verfahren, das eine Kohlenwasserstoff-Wasser-Trenneinheit, eine Öl-Gas-Trenneinheit, eine Prozessdampferzeugungseinheit und eine Methanolrückgewinnungseinheit umfasst. Die Kohlenwasserstoff-Wasser-Trenneinheit ist so konfiguriert, dass sie einen Strom trennt, der ein MTP-Reaktionsprodukt und einen Verdünnungsdampf enthält, um einen gasförmigen Kohlenwasserstoff, eine schwere Kohlenwasserstoffkomponente und kondensiertes Wasser zu erhalten, aus dem eine schwere Kohlenwasserstoffkomponente abgetrennt wird. Die Öl-Gas-Trenneinheit ist so konfiguriert, dass sie das in dem gasförmigen Kohlenwasserstoff mitgeführte Wasser und die schwere Kohlenwasserstoffkomponente, die durch die Trennung der Kohlenwasserstoff-Wasser-Trenneinheit erhalten wurde, trennt, um eine Gasphasenkomponente, ein Kohlenwasserstoffkondensat und Restwasser zu erhalten. Die Prozessdampferzeugungseinheit ist so konfiguriert, dass sie mindestens einen Teil des kondensierten Wassers extrahiert, das von der schweren Kohlenwasserstoffkomponente abgetrennt wurde, die durch Trennen der Kohlenwasserstoff-Wasser-Trenneinheit erhaltenen schweren Kohlenwasserstoffkomponente abgetrennt wird, um eine Kohlenwasserstoffkomponente darin zu extrahieren und eine Extraktion zu erhalten. Das Wasser der Kohlenwasserstoffkomponente wird auch zur Behandlung des Wassers verwendet, aus dem die Kohlenwasserstoffkomponente extrahiert wurde, um einen Prozessdampf zu erhalten, in dem die salzhaltigen Tröpfchen abgetrennt werden. Die Methanolrückgewinnungseinheit ist so konfiguriert, dass sie mindestens einen Teil des kondensierten Wassers behandelt, das von der schweren Kohlenwasserstoffkomponente abgetrennt wird, die durch Trennen der Kohlenwasserstoff-Wasser-Trenneinheit erhaltenen schweren Kohlenwasserstoffkomponente abgetrennt wird, um Methanol, DME und darin enthaltene leichte Komponenten von nicht kondensiertem Gas abzutrennen.

Die Druckschrift WO 93/12200 offenbart wird ein Verfahren zum Quenchen des Abflusses von Kohlenwasserstoff-Pyrolyseeinheiten und zum Entfernen der dabei entstehenden Schweröle und Teere, um deren Ansammlung im rückgeführten Quenchwasser und im überschüssigen Quenchwasser zu verhindern. Das gasförmige Pyrolyseabgas wird zunächst auf eine Zieltemperatur nahe oder gleich dem Taupunkt von Wasser bei dem im Abgasstrom herrschenden Druck abgekühlt. Das gasförmige Abgas wird dann beim Eintritt in einen Abscheidebehälter verdampft. Die Bedingungen im Abscheidebehälter sind so, dass alle Schweröle und Teere kondensieren und als im Wesentlichen wasserfreies Konzentrat aus dem System entfernt werden. Nach der anfänglichen Abkühlung ist der gasförmige Abstrom weitgehend frei von Schwerölen und Teeren, die eine stabile Öl/Wasser-Emulsion bilden können, und der Abfluss wird mit Wasser gequencht, um den den Abkühlungsprozess abzuschließen. Durch die Verhinderung der Bildung einer stabilen Öl/Wasser-Emulsion bei der Wasserabschreckung des gasförmigen Abwassers, werden die mit einem großen Volumenstrom von ölverschmutztem Wasser verbundenen Entsorgungsprobleme beseitigt. Darüber hinaus wird die Verschmutzung im dem rezirkulierenden Quenchwasserkreislauf und dem System für überschüssiges Quenchwasser reduziert. Das beschriebene Verfahren ist besonders geeignet zur Entfernung von Schwerölen und Teeren, die bei der Pyrolyse von mittelschweren Kohlenwasserstoffen, wie Flüssiggas und leichten Naphtha Erdölgase und leichte Naphthas, entstehen.

Zusammenfassend ergeben sich folgende Nachteile bei Quenchsystemen gemäß Stand der Technik:
(a) Entweder es wird keine dedizierte Kohlenwasserstoff-Wasser-Phasentrennung offenbart oder aber der betreffende Phasenabscheider weist ein großes Volumen auf, weil der komplette Quenchwasserkreislauf durch den Phasenabscheider geführt werden muss.
(b) Bei der weiteren Verarbeitung der abgetrennten Kohlenwasserstofffraktion kommt es zu einem Vermischen der schweren Kohlenwasserstoffe mit leichten Kohlenwasserstoffen in der stromabwärts angeordneten Fraktionierungssektion, umfassend einen Dreiphasenabscheider und üblicherweise mehrere Destillationskolonnen, was zu einer Wiederholung der Abtrennung schwerer Kohlenwasserstoffe von den leichten Kohlenwasserstoffen in der Reinigung führt.

Neben dem zusätzlichen Energieaufwand müssen die Apparate auch entsprechend größer ausgelegt werden, was die Investitionskosten einer entsprechenden Anlage erhöht. Dieses Problem intensiviert sich noch dadurch, dass die Fraktionierungssektion üblicherweise unter Überdruck betrieben wird und daher entsprechend ausgelegt werden muss.

### Beschreibung der Erfindung

Insgesamt besteht daher weiterhin Bedarf an einem einfachen und vorteilhaften Verfahren zum Quenchen eines Wasser und Kohlenwasserstoffe enthaltenden, fluiden Produktstroms aus einem Synthesereaktor. Die Aufgabe der Erfindung ist es daher, ein solches Verfahren bereitzustellen.

Diese Aufgabe wird im Wesentlichen durch ein Verfahren mit den Merkmalen des Anspruchs 1 gelöst. Weitere, insbesondere bevorzugte Ausgestaltungen des erfindungsgemäßen Verfahrens finden sich in den Unteransprüchen der jeweiligen Kategorie.

Als Aufreinigungsschritte oder Trennschritte sind im Zusammenhang mit der vorliegenden Erfindung grundsätzlich alle Verfahrensschritte zu betrachten, die sich eines thermischen Trennverfahrens bedienen; bevorzugt wird hierbei die Destillation oder Rektifikation verwendet.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem anderen der beiden Bereiche strömen kann, unbeachtlich etwaiger zwischengeschalteter Bereiche, Bauteile oder benötigter Fördermittel. Unter einem Fluid oder fluiden Medium werden dabei Substanzen verstanden, die sich unter dem Einfluss von Scherkräften kontinuierlich verformen, also fließen. Insbesondere sind dies Gase und Flüssigkeiten, aber auch mehrphasige Flüssig-Flüssig-Gemische und Gas-Flüssig-Gemische, wie beispielsweise Gasströmungen mit mitbewegtem Kondensatanteil oder Aerosole.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile, in Betracht ziehen, die ihm aufgrund seines Fachwissens zur Durchführung dieses Verfahrensschrittes notwendig oder sinnvoll erscheinen.

Unter Oxygenaten werden grundsätzlich alle sauerstoffhaltigen Kohlenwasserstoffverbindungen verstanden, die sich unter Oxygenatumsetzungsbedingungen zu Olefinen, insbesondere zu kurzkettigen Olefinen wie Propylen, und weiteren Kohlenwasserstoffprodukten umsetzen lassen.

Als kurzkettige Kohlenwasserstoffe werden im Rahmen der vorliegenden Erfindung insbesondere die Kohlenwasserstoffe verstanden, die bei Umgebungsbedingungen gasförmig vorliegen, beispielsweise, im Falle der Olefine, Ethylen, Propylen sowie die isomeren Butene 1-Buten, cis-2-Buten, trans-2-Buten, iso-Buten.

Als höhere Kohlenwasserstoffe werden im Rahmen der vorliegenden Erfindung insbesondere diejenigen Kohlenwasserstoffe verstanden, die bei Umgebungsbedingungen flüssig vorliegen.

Die genannten Aggregatzustände fest, flüssig und gasförmig bzw. dampfförmig sind immer in Bezug auf die lokalen physikalischen Bedingungen zu verstehen, die bei dem jeweiligen Verfahrensschritt oder in dem jeweiligen Anlagenteil herrschen, sofern nichts anderes angegeben ist. Im Rahmen der vorliegenden Anmeldung sind die Aggregatzustände gasförmig bzw. dampfförmig als synonym zu betrachten. Die Bezeichnung "dampfförmig" dient lediglich zur Verdeutlichung, dass der betreffende Stoff bei Umgebungsbedingungen normalerweise flüssig vorliegt.

Unter einem Auftrennen eines Stoffstroms ist im Zusammenhang mit der vorliegenden Erfindung eine Aufteilung des Stroms in mindestens zwei Teilströme zu verstehen. Wenn nichts anderes angegeben wird, ist davon auszugehen, dass die stoffliche Zusammensetzung der Teilströme derjenigen des Ausgangsstroms entspricht, außer für die Fälle, bei denen dem Fachmann unmittelbar einsichtig ist, dass eine Änderung der stofflichen Zusammensetzung der Teilströme infolge der Bedingungen der Auftrennung zwangsweise auftreten muss, wie dies beispielsweise bei der Destillation der Fall ist.

Unter einer Benzinfraktion ist ein überwiegend, bevorzugt weitgehend vollständig aus höheren Kohlenwasserstoffen bestehendes, unter Umgebungsbedingungen flüssig vorliegendes Stoffgemisch zu verstehen, das geeignet sein kann, als Ottokraftstoff verwendet zu werden.

Unter dem überwiegenden Teil einer Fraktion, eines Stoffstroms etc. ist ein Anteil zu verstehen, der mengenmäßig größer ist als alle anderen jeweils für sich betrachteten Anteile. Insbesondere bei binären Gemischen oder bei der Auftrennung einer Fraktion in zwei Teile ist darunter ein Anteil von mehr als 50 Gew.-% zu verstehen, sofern in konkreten Fall nichts anderes angegeben ist.

Unter der Angabe, dass ein Stoffstrom überwiegend aus einer Komponente oder Komponentengruppe besteht, ist zu verstehen, dass der Stoffmengenanteil (Molenbruch) oder Massenanteil (Massenbruch) dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Massenanteil (Massenbruch) zugrunde gelegt.

Erfindungsgemäß wird ein Wasser und Kohlenwasserstoffe enthaltender Produktstrom aus einem Synthesereaktor in einem mindestens zwei Quenchstufen umfassenden Quenchsystem gekühlt. Dabei wird zuerst der Produktstrom des Synthesereaktors in der ersten Quenchstufe mit einem Wasser enthaltenden ersten Quenchmedium in Kontakt gebracht, wobei eine erste Quenchfraktion und ein erster Abflussstrom erhalten werden. Dieser erste Abflussstrom wird anschließend in einer zweiten Quenchstufe mit einem zweiten, Wasser enthaltenden Quenchmedium in Kontakt gebracht, wobei eine zweite Quenchfraktion und ein zweiter Abflussstrom erhalten werden. Letzterer kann nachfolgend beispielsweise einer weiteren Kohlenwasserstofffraktionierung zugeführt werden. Bevorzugt wird der zweite Abflussstrom gasförmig aus der zweiten Quenchstufe ausgeleitet. Auch in diesem Fall kann der zweite Abflussstrom noch Flüssigkeitsanteile umfassen, beispielsweise in Form mitgerissener Flüssigkeitsanteile oder als Aerosole.

Ein Teil der ersten Quenchfraktion wird mit der zweiten Quenchfraktion zu einem Gesamtstrom kombiniert. Dieser Gesamtstrom wird mindestens einem ersten Phasenabscheider zugeführt und dort in einen dritten, überwiegend Kohlenwasserstoffe umfassenden, flüssigen Abflussstrom und eine wässrige Fraktion aufgetrennt. Wenigstens ein erster Teil der wässrigen Fraktion wird als zweites Quenchmedium in die zweite Quenchstufe zurückgeführt.

Entscheidend ist es, dass nicht mehr beide Quenchfraktionen der zwei Quenchstufen in einen gemeinsamen Phasenabscheider gelangen und es somit nicht mehr zur Bildung einer Mischtemperatur an einer Stelle kommt, an der verhältnismäßig viele leicht verdampfbare Kohlenwasserstoffe enthalten sind. Durch die Verlegung der Phasentrennung in den kälteren Quenchkreislauf werden diese Kohlenwasserstoff sicher in der flüssigen Phase gehalten und können auf diese Weise in einem Kohlenwasserstoff-Flüssigprodukt gesammelt werden, ohne eine zusätzliche, erneute Fraktionierung zu durchlaufen. Dabei besteht auch die Möglichkeit, die Temperatur des Quenchmediums in der ersten Quenchstufe so einzustellen, dass die Kohlenwasserstoffkondensation in der ersten Quenchstufe minimiert ist und weitgehend nur in der zweiten Quenchstufe erfolgt.

Gleichermaßen ist es so möglich, die Betriebsbedingungen für die Phasentrennung durch Verlagerung des Phasenabscheiders in den Kaltbereich des Quenchsystems zu optimieren. Der überwiegende Teil der Kohlenwasserstoffe wird nun erst in der zweiten Quenchstufe kondensiert. Simulationsrechnungen zeigen, dass der im Phasenabscheider behandelte Massenstrom damit um den Faktor sechs reduziert werden kann. Damit werden die Investitionskosten signifikant reduziert, da sich die Volumina der betreffenden Apparate und Behälter entsprechend verkleinern.

Bei dem herkömmlichen Quenchverfahren gemäß Stand der Technik wird das Reaktorproduktgas des Synthesereaktors, das ggf. bereits zur Wärmeintegration ohne Kondensation vorgekühlt wurde, in ein zweistufiges Quenchsystem eingeleitet. Hier findet eine weitere, schlagartige Abkühlung durch Inkontaktbringen mit kaltem Wasser als Quenchmedium statt, die zur Kondensation der im Reaktorproduktstrom enthaltenen, höhersiedenden Komponenten führt; dies sind beispielsweise Wasser, Alkohole und höhersiedende Kohlenwasserstoffe mit höherer Molmasse.

Das herkömmliche, zweistufige Quenchsystem verwendet einen gemeinsamen Quenchwasserkreislauf für beide Löschstufen, obwohl die beiden Quenchstufen bei unterschiedlichen Temperaturen betrieben werden.

Das erfindungsgemäße Verfahrensschema verwendet dagegen zwei getrennte Quenchwasserkreisläufe für die zwei Quenchstufen, die mit unterschiedlichen Quenchwassertemperaturen betrieben werden. Das hat mehrere Vorteile:
(1) Die zur Durchführung der Flüssig-Flüssig-Trennung benötigten Apparate und Behälter sind wesentlich kleiner als bei dem Quenchverfahren gemäß Stand der Technik, da erfindungsgemäß in dem zugehörigen Phasenabscheider nur eine kleine Wassermenge behandelt werden muss, die sich im Wesentlichen aus dem Quenchwasserkreislauf der zweiten Quenchstufe und in der ersten Quenchstufe noch nicht kondensierten Wasseranteilen zusammensetzt. Der große Quenchwasserkreislauf der ersten Quenchstufe wird nur in einem optionalen und - falls vorhanden - kleinen Phasenvorabscheider behandelt, um die Ansammlung von Kohlenwasserstoffen in diesem Kreislauf zu vermeiden.
(2) Ein Verdampfen bzw. Ausgasen der in der zweiten Quenchstufe im Phasenabscheider kondensierten Komponenten wird vermieden, da das heißere Quenchmedium der ersten Quenchstufe nicht mit dem kühlen Quenchmedium der zweiten Quenchstufe gemischt wird.
(3) Die Phasentrennung von Kohlenwasserstoffen und Wasser ist bei kalten Temperaturen einfacher, da die Löslichkeit von Kohlenwasserstoffen in Wasser verringert wird und die Grenzflächenspannung erhöht wird, was eine bessere Trennung ermöglicht.
(4) Die im Phasenabscheider des Quenchsystems kondensierten Kohlenwasserstoffe sind innerhalb des Produktspektrums der vorgelagerten Synthesereaktion die schwersten Kohlenwasserstoffe mit dem höchsten Siedepunkt. Diese Kohlenwasserstoffe können direkt, also unter Umgehung der weiteren Auftrennung bzw. Fraktionierung des Kohlenwasserstoffprodukts, zu einer als Benzinprodukt bezeichneten Kohlenwasserstofffraktion mit höherer Molmasse und höherem Siedepunkt geleitet werden. Insbesondere umfasst das Benzinprodukt die bei Umgebungsbedingungen flüssigen Kohlenwasserstoffe. Gemäß des herkömmlichen Quenchverfahrens würde ein größerer Anteil der höhersiedenden Kohlenwasserstofffraktion in die weitere Auftrennung bzw. Fraktionierung des Kohlenwasserstoffprodukts gelangen, was zu einer Wiederholung der Trennung der schwerersiedenden Kohlenwasserstoffe von den leichtersiedenden Kohlenwasserstoffen führt. Zudem steigt durch die Anwesenheit schwerersiedender Kohlenwasserstoffe in den Trennapparaten der Fraktionierungssektion die Gefahr der Bildung von Ablagerungen und Verstopfungen (sog. Fouling bzw. Gum-Bildung).
(5) Das Temperaturprofil in der ersten Quenchstufe kann durch Regelung der Temperatur und des Volumenstroms des wasserhaltigen ersten Quenchmediums so eingestellt werden, dass die Kondensation schwerersiedender Kohlenwasserstoffe in der ersten Quenchstufe vermieden und stattdessen in die zweite Quenchstufe verschoben wird.

### Bevorzugte Ausgestaltungen der Erfindung

Eine bevorzugte Ausgestaltung des erfindungsgemäßen Verfahrens ist dadurch gekennzeichnet, dass die Eintrittstemperatur des ersten Quenchmediums in die erste Quenchstufe höher ist als die Eintrittstemperatur des zweiten Quenchmediums in die zweite Quenchstufe. Dies trägt dazu bei, dass in der erste Quenchstufe vor allem Wasser und nur in geringem Ausmaß auch Kohlenwasserstoffe kondensiert werden. Letztere werden in der zweiten, kühleren Quenchstufe kondensiert, wodurch deren Abscheidung und die nachfolgende Phasentrennung erleichtert wird.

Besonders bevorzugt ist es dabei, dass die Eintrittstemperatur des ersten Quenchmediums in die erste Quenchstufe so eingestellt wird, dass die erste Quenchfraktion zu mindestens 70 Gew.-%, bevorzugt zu mindestens 80 Gew.-%, meist bevorzugt mindestens 90 Gew.-% aus Wasser besteht. Auf diese Weise kann ein etwaiger zweiter Phasenabscheider zur Abtrennung einer Kohlenwasserstoffphase minimiert werden.

Eine bevorzugte Ausgestaltung des erfindungsgemäßen Verfahrens ist dadurch gekennzeichnet, dass die Temperatur des zweiten Teils der ersten Quenchfraktion geringer als die Austrittstemperatur der zweiten Quenchfraktion ist. Dies kann durch entsprechendes Kühlen der ersten Quenchfraktion, zumindest aber des zweiten Teils der ersten Quenchfraktion, gewährleistet werden. Hiermit wird sichergestellt, dass eine Erwärmung des gebildeten Gesamtstroms und somit ein Ausgasen von Kohlenwasserstoffen im ersten Phasenabscheider unterbleibt.

Bevorzugt wird der erste Teil der ersten Quenchfraktion vor der Rückführung in die erste Quenchstufe auf eine Temperatur zwischen 20 und 70 °C abgekühlt und/oder dass der zweite Teil der ersten Quenchfraktion vor Eintritt in die zweite Quenchstufe des ersten Quenchmediums auf eine Temperatur zwischen 30 und 50 °C gekühlt. Untersuchungen haben gezeigt, dass bei Einhalten dieser Temperaturbereiche die zuvor geschilderten Vorteile in besonders ausgeprägter Weise erhalten werden

In einem weiteren Aspekt des erfindungsgemäßen Verfahrens wird ein erster Teil der wässrigen Fraktion als zweites Quenchmedium zurück in die zweite Quenchstufe geführt wird und ein zweiter Teil der wässrigen Fraktion einer weiteren Aufreinigung zugeführt wird. Durch das Ausleiten des zweiten Teils der wässrigen Fraktion als Spülstrom oder Purgestrom wird eine Anreicherung unerwünschter Komponenten im Quenchmedium-Kreislauf der zweiten Quenchstufe verhindert. Eine Anreicherung bestimmter, insbesondere polarer organischer Komponenten wie Alkohole, Ether oder Ketone könnte wegen ihrer lösungsvermittelnden Eigenschaften ansonsten die Phasentrennung im ersten Phasenabscheider behindern.

Besonders bevorzugt ist dabei die weitere Aufreinigung eine Kolonne zur Rückgewinnung von Oxygenaten und/oder eine Wasserreinigung. Auf diese Weise können die Störkomponenten nicht nur aus dem Wasserkreislauf entfernt, sondern zudem auch wiedergewonnen und ggf. in den Synthesereaktor zurückgeführt werden.

Bevorzugt wird der fluide Produktstrom nach dem Austritt aus dem Synthesereaktor und vor Eintritt in die erste Quenchstufe des mehrstufigen Quenchsystems abgekühlt. Die Abkühlung kann dabei mit kalten Prozessmedien erfolgen, die dabei erwärmt werden, was die Wärmeintegration des Gesamtverfahrens verbessert. Die Abkühlung sollte hierbei nur soweit erfolgen, dass noch kein Kondensat anfällt, also die Temperatur des fluiden Produktstroms auch nach der Abkühlung immer noch oberhalb des Taupunkts liegt. Somit können die verwendeten Wärmetauscher konstruktiv einfach gehalten werden und eine Kondensatabtrennung entfällt. Ferner ist das in der ersten Quenchstufe auftretende Temperaturfenster enger.

Ein weiterer Aspekt des erfindungsgemäßen Verfahrens ist dadurch gekennzeichnet, dass der dritte Abflussstrom, der mindestens einen Teil der im Quenchsystem kondensierten Kohlenwasserstoffe enthält, vollständig aus dem Quenchsystem abgeführt wird. Dies verringert den Mengenstrom der in einem nachgeschalteten Verfahren zur Kohlenwasserstofffraktionierung zu behandelnden Kohlenwasserstoffgemische und es wird vermieden, eine Trennung leichtsiedender Kohlenwasserstoffe von Schwersiedern, die im dritten Abflussstrom enthalten sind, zu wiederholen.

Bevorzugt erfolgt die Rückführung des ersten Teils der ersten Quenchfraktion in die ersten Quenchstufe mittels Durchflussregelung und/oder dass die Rückführung des ersten Teils der wässrigen Fraktion als zweites Quenchmedium in die zweite Quenchstufe mittels Niveauregelung. Untersuchungen haben ergeben, dass diese Regelungskonzepte besonders praktikabel und effizient sind. Besonders die Kombination der beiden Regelungsarten stellt sicher, dass die auch die Temperaturregelung an dem in dem erfindungsgemäßen Verfahren sensitiven Punkt der ersten Quenchstufe optimiert ist.

Gemäß einer besonders bevorzugten Ausgestaltung des erfindungsgemäßen Verfahrens wird die erste Quenchfraktion nach dem Ausleiten aus der ersten Quenchstufe abgekühlt und in einen zweiten Phasenabscheider geleitet, wobei eine wasserreiche Flüssigphase erhalten wird, die als erster Teil der ersten Quenchfraktion zu der ersten Quenchstufe zurückgeführt wird und wobei eine kohlenwasserstoffreiche Flüssigphase erhalten wird, die als zweiter Teil der ersten Quenchfraktion mit der zweiten Quenchfraktion aus der zweiten Quenchstufe zu dem Gesamtstrom kombiniert wird. Da es bevorzugt wird, in der ersten Quenchstufe weitgehend nur Wasser abzuscheiden, wird hiermit verhindert, dass sich Kohlenwasserstoffe im Quenchmedien-Kreislauf der ersten Quenchstufe anreichern.

Bevorzugt wird der zweite Abflussstrom einer bevorzugt mehrstufigen Kohlenwasserstofffraktionierungsvorrichtung zugeführt und in dieser in olefinhaltige Kohlenwasserstofffraktionen aufgetrennt. Auf diese Weise können bestimmte Kohlenwasserstofffraktionen oder Reinprodukte gewonnen werden. Ferner hat es sich herausgestellt, dass der zweite Abflussstrom aus der zweiten Quenchstufe bei der erfindungsgemäßen Verfahrensführung einen Großteil der als Wertprodukt begehrten Olefine enthält. Es ist daher günstig, diesen zweiten Abwasserstrom, vorzugsweise verdichtet, einer Olefingewinnung zuzuführen.

### Ausführungsbeispiel

Weitere Merkmale, Vorteile und Anwendungsmöglichkeiten der Erfindung ergeben sich aus der nachfolgenden Beschreibung eines Ausführungsbeispiels und der Zeichnung. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbezügen.

Es zeigen:
- Fig. 1: das grundsätzliche Fließschema eines Quenchverfahrens nach dem Stand der Technik und
- Fig. 2: das grundsätzliche Fließschema eines Quenchverfahrens gemäß einer Ausgestaltung der Erfindung.

Figur 1 wurde bereits bei der Diskussion des Standes der Technik ausführlich erörtert.

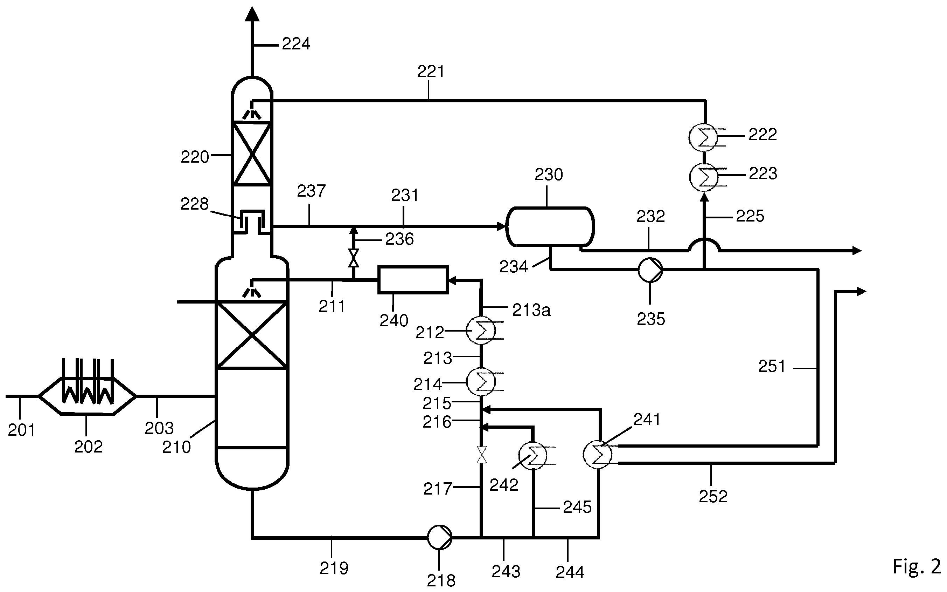

Fig. 2 zeigt schematisch eine mögliche Ausgestaltung des erfindungsgemäßen Quenchverfahrens mit zwei Quenchstufen 210 und 220. In der vorliegenden, beispielhaften Ausgestaltung sind beide Quenchstufen senkrecht übereinander in einem gemeinsamen Behälter angeordnet. Dies hat den Vorteil der Platzersparnis an eng begrenzten Aufstellungsorten. Möglich ist aber auch eine Ausgestaltung mit zwei getrennten Behältern für die beiden Quenchstufen. Hierdurch wird eine flexiblere Aufstellung des Quenchsystems ermöglicht, beispielsweise wenn die Bauhöhe limitiert ist.

Der Produktstrom aus dem Synthesereaktor wird über Leitung 201 in den Wärmetauscher 202 und von dort über Leitung 203 weiter in die erste Quenchstufe 210 geführt. Dort wird er mit einem Wasser enthaltenden ersten Quenchmedium in Kontakt gebracht, welches über Leitung 211 eingedüst wird. Der entstehende Flüssigkeitsstrom wird als erste Quenchfraktion über Leitung 219 ausgeleitet. Der erhaltene, gasförmige erste Abflussstrom verlässt die erste Quenchstufe nach oben hin und tritt durch den Kaminboden 228 in die zweite Quenchstufe ein. Der in letzterer erhaltene, gasförmige zweite Abflussstrom verlässt die zweite Quenchstufe über Leitung 224 und wird optional einer weiteren, nicht bildlich dargestellten Aufarbeitung zugeführt, die zumeist eine mehrstufige Destillation zur Gewinnung mehrerer Kohlenwasserstoffproduktfraktionen umfasst, darunter leichte Produktfraktionen wie Ethylen und Propylen.

Die erste Quenchfraktion wird anschließend über Leitungen 217, 216, 215 und 213 mittels Pumpe 218 in Leitung 211 zurückgeführt, wobei sie zusätzlich abgekühlt wird. Dies kann in der dargestellten Weise mit zwei Wärmetauschern 212, 214 geschehen, wobei Wärmetauscher 214 im vorliegenden Beispiel als Luftkühler ausgestaltet ist; grundsätzlich ist aber jede Kühlung denkbar.

Nach dem Abkühlen der ersten Quenchfraktion auf die für die erste Quenchstufe erforderliche Temperatur des Quenchmediums von beispielsweise 50 °C kann optional auch ein Phasenabscheider 240 eingesetzt werden, um Kohlenwasserstoffansammlungen im ersten Quenchwasserkreislauf zu vermeiden. Die Trennung von Kohlenwasserstoffen und Wasser erfolgt aufgrund der Löslichkeiten auch hier im kalten Bereich des Quenchmittelkreislaufs effizienter als im heißen Strom, der den Boden der ersten Quenchstufe 210 verlässt. Eine vollständige Trennung von Wasser und Kohlenwasserstoffen ist in der ersten Quenchstufe 210 und der zugehörigen Quenchmittelrezirkulierung nicht erforderlich. Im Sinne der Erfindung ist es vielmehr wichtig, dass ein Großteil des enthaltenen oder als Quenchmedium zugegebenen Wassers in der ersten Quenchstufe abgeschieden wird, ohne dass es gleichzeitig zu einer weitgehenden Abscheidung von Kohlenwasserstoffen kommt.

Eine Möglichkeit zur Ausgestaltung des optionalen Flüssig-Flüssig-Phasenabscheiders 240 kann beispielsweise eine vergrößerte horizontale Rohrgröße für eine bestimmte Länge sein, um laminare Strömungsverhältnisse und dadurch bedingt die Ansammlung von Kohlenwasserstoffen als spezifisch leichtere Flüssigphase im oberen Teil des Rohres zu ermöglichen. In dieser Ausgestaltung befindet sich dieses vergrößerte Rohr nachgeschaltet zu den Wärmetauschern 212 und 214 und vor einem Regelventil für die Zufuhr des Quenchmediums zu der ersten Quenchstufe 210 (in Leitung 211 und 236 angedeutet). In die Unterseite eines entsprechend vergrößerten Rohres als Phasenabscheider 240 wird eine Ableitung zur Abtrennung einer wässrigen Phase installiert, die sodann als erstes Quenchmedium zu der ersten Quenchstufe zugeführt wird. Über eine auf der Oberseite des Phasenabscheiders 240 vorzugsweise angeordnete Ableitung wird eine kohlenwasserstoffreiche Flüssigphase ausgeleitet.

Die im optionalen Flüssig-Flüssig-Phasenabscheider 240 abgetrennte kohlenwasserstoffreiche Phase kann mittels Leitung 236 und dem in dieser Leitung angebrachten, in der Figur angedeuteten Regelventil zu Leitung 231 geführt und in diese eingebracht werden, die einen Teil des Quenchmedium-Kreislaufs der zweiten Quenchstufe 220 bildet. Gegebenenfalls kann es sinnvoll sein, mittels eines in den Leitungsweg 236 integrierten, zusätzlichen und bildlich nicht dargestellten Kühler die abgetrennte kohlenwasserstoffreiche Phase vor ihrem Einbringen in Leitung 231 weiter abzukühlen. Der Durchfluss dieser kohlenwasserstoffreichen Phase zu Leitung 231 kann so eingestellt werden, dass ferner auch überschüssiges Wasser aus der ersten Quenchstufe 210 über diesen Weg in die zweite Quenchstufe 220 überführt wird. Auf diese Weise wird das Quenchmedium-Inventar der ersten Quenchstufe konstant gehalten und die kohlenwasserstoffreiche Phase wird vollständig in die zweite Quenchstufe 220 überführt.

Die kohlenwasserstoffarme Wasserphase wird aus dem optionalen Phasenabscheider 240 über Leitung 211 zurück in die erste Quenchstufe 210 geführt. Der Quenchmedium-Kreislauf der zweiten Quenchstufe 220 wird aus den Leitungen 237, 231, dem Phasenabscheider 230, Leitungen 234, 225, 221 und Pumpe 235 gebildet. Über Wärmetauscher 222 und 223 kann das Quenchmedium vor seiner Rückführung in die zweite Quenchstufe abgekühlt werden. Über Leitungen 251 und 252 kann überschüssiges Wasser aus dem Quenchsystem ausgeleitet und ggf. mittels Wärmetauscher 241 zum Vorkühlen des Quenchmedium-Kreislaufs der ersten Quenchstufe verwendet werden. Bevorzugt wird das aus dem Quenchsystem ausgeleitete, wässrige Quenchmedium einer nicht gezeigten Methanol-Rückgewinnungskolonne zugeführt, um in diesem gelöste Oxygenate wie Methanol oder DME zurückzugewinnen.

Falls die erste Quenchstufe 210 so betrieben wird, dass in ihr keinerlei Kohlenwasserstoffe abgeschieden werden, kann auf den Phasenabscheider 240 verzichtet werden. Dann wird die erste Quenchfraktion nach Abkühlung als erstes Quenchmedium über Leitung 213 bzw. 211 zu der ersten Quenchstufe zurückgeführt und dieser über Leitung 211 aufgegeben. Trotzdem wird ein zweiter Teil der ersten Quenchfraktion über Leitung 236 in den Quenchmedium-Kreislauf der zweiten Quenchstufe 220 eingeführt, um überschüssiges Wasser über Leitungen 251 und 252 auszuschleusen. Außerdem werden im zweiten Teil der ersten Quenchfraktion gelöste Kohlenwasserstoffe unter den kühleren Bedingungen der zweiten Quenchstufe als organische Phase abgeschieden und mittels des Phasenabscheiders 230 abgetrennt.

Die in dem Phasenabscheider 230 abgetrennten und über Leitung 232 ausgeleiteten Kohlenwasserstoffe können grundsätzlich in den Synthesereaktor, beispielsweise den OTO-Reaktor, zurückgeführt oder weiter aufbereitet werden. Besonders günstig ist es, diese Fraktion nach optionaler Behandlung in einer Destillationskolonne zur Entfernung von Leichtsiedern, dem Benzinprodukt einer OTO-Synthese zuzusetzen.

Die Gesamtmenge des gemäß der Erfindung zu behandelnden, wässrigen Quenchmediums im Phasenabscheider 230 ist im Vergleich zum herkömmlichen Quenchsystem mit gemeinsamem Phasenabscheider für zwei Quenchstufen deutlich reduziert. Simulationsrechnungen, bei denen die in Fig. 1 und Fig. 2 dargestellten Quenchsysteme miteinander verglichen werden, zeigen hier eine bis zu sechs Mal kleinere Gesamtmenge des zu behandelnden, wässrigen Quenchmediums, was zu entsprechend kleineren Apparategrößen aller Apparate im zweiten Quenchmedium-Kreislauf und damit zu erheblichen Einsparungen bei den Investitionskosten führt.

Zudem wird mit der Erfindung die Effizienz der Flüssig-Flüssig-Phasentrennung und -Abscheidung verbessert, da die Betriebstemperatur des Phasenabscheiders niedriger als im herkömmlichen System gemäß Fig. 1 ist. Bei reduzierter Betriebstemperatur ist die Grenzflächenspannung zwischen Wasser und Kohlenwasserstoffphase erhöht, was zu einer schnelleren Tröpfchenbildung mit erhöhter Tröpfchengröße führt. Überdies ist die Löslichkeit von Kohlenwasserstoffen in Wasser bei kälteren Temperaturen reduziert, was die Effizienz der Phasenseparation weiter erhöht. Die Betriebsbedingungen, insbesondere die abgesenkte Temperatur für die Flüssig-Flüssig-Phasentrennung, ermöglichen somit eine effizientere Phasentrennung und die Anlagengröße wird weiter reduziert. Gegebenenfalls kann stromaufwärts des Phasenabscheiders 230 ein weiterer, nicht gezeigter Wärmetauscher angeordnet werden, um die zweite Quenchfraktion vor Eintritt in den Phasenabscheider abzukühlen und die Phasentrennung weiter zu unterstützen.

Im erfindungsgemäßen Verfahren hat der über Leitung 251 vom Phasenabscheider 230 zur nicht dargestellten Methanol-Rückgewinnungskolonne gepumpte Strom eine niedrige Temperatur. Diese Temperatur kann durch Wärmeintegration mit dem heißen Teil des ersten Quenchwasserkreislaufs erhöht werden. Dazu dient dieser Strom dem Wärmetauscher 241 als Wärmeübertragungsmittel. Diese Wärmeintegration steht in Konkurrenz zur aus dem Stand der Technik bereits bestehenden Wärmeintegration mit Rückflussströmen aus nachgeschalteten Destillationskolonnen. Besonders günstig ist daher eine Parallelverschaltung von zwei Wärmetauschern 241 und 242, bei denen Wärmetauscher 241 in beschriebener Weise mit dem Abwasserstrom des zweiten Quenchmediums-Kreislaufs und Wärmetauscher 242 mit einer nicht dargestellten Destillationskolonne verbunden ist.

### Bezugszeichenliste:

- 101: Leitung
- 110: erste Quenchstufe
- 111 - 116: Leitung
- 120: zweite Quenchstufe
- 121 - 124: Leitung
- 126: Wärmetauscher
- 130: Phasenabscheider
- 133 - 134: Leitung
- 135: Pumpe
- 136 - 145: Leitung
- 146: Wärmetauscher

- 201: Leitung
- 202: Kühler
- 203: Leitung
- 210: erste Quenchstufe
- 211: Leitung
- 212: Wärmetauscher
- 213 - 213a: Leitung
- 214: Wärmetauscher
- 215 - 217: Leitung
- 218: Pumpe
- 219: Leitung
- 220: zweite Quenchstufe
- 221: Leitung
- 222 - 223: Wärmetaucher
- 224 - 225: Leitung
- 228: Kaminboden
- 230: erster Phasenabscheider
- 231 - 234: Leitung
- 235: Pumpe
- 236 - 237: Leitung
- 240: zweiter Phasenabscheider
- 241 - 242: Wärmetauscher
- 243 - 245: Leitung
- 251 - 252: Leitung

## Patentansprüche

1. Verfahren zum Quenchen eines Wasser und Kohlenwasserstoffe enthaltenden, fluiden Produktstroms aus einem Oxygenat-zu-Olefin-(OTO)-Synthesereaktor, in einem mehrere Quenchstufen umfassenden Quenchsystem, umfassend die folgenden Schritte:
(a) Einleiten des Produktstroms in eine erste Quenchstufe, Inkontaktbringen des Produktstroms in der ersten Quenchstufe mit einem Wasser enthaltenden ersten Quenchmedium zur Bildung einer ersten Quenchfraktion und eines ersten Abflussstroms, Ausleiten der ersten Quenchfraktion und des ersten Abflussstroms aus der ersten Quenchstufe, Rückführen eines ersten Teils der ersten Quenchfraktion zu der ersten Quenchstufe als erstes Quenchmedium,
(b) Einleiten des ersten Abflussstroms in eine zweite Quenchstufe, Inkontaktbringen des ersten Abflussstroms in der zweiten Quenchstufe mit einem Wasser enthaltenden zweiten Quenchmedium zur Bildung einer zweiten Quenchfraktion und eines zweiten Abflussstroms, Ausleiten der zweiten Quenchfraktion und des zweiten Abflussstroms aus der zweiten Quenchstufe, wobei
(c) ein zweiter, nicht zu der ersten Quenchstufe zurückgeführter Teil der ersten Quenchfraktion mit mindestens einem Teil der zweiten Quenchfraktion aus der zweiten Quenchstufe zu einem Gesamtstrom kombiniert wird und dass
(d) dieser Gesamtstrom wenigstens einem ersten Phasenabscheider zugeführt wird, in dem der Gesamtstrom in einen dritten, überwiegend Kohlenwasserstoffe umfassenden, flüssigen Abflussstrom und eine wässrige Fraktion aufgetrennt wird, und
(e) dass mindestens ein erster Teil der wässrigen Fraktion als zweites Quenchmedium nach Schritt (b) zurückgeführt wird, wobei
(f) die Eintrittstemperatur des ersten Quenchmediums in die erste Quenchstufe so eingestellt wird, dass die erste Quenchfraktion zu mindestens 70 Gew.-%, bevorzugt zu mindestens 80 Gew.-%, meist bevorzugt mindestens 90 Gew.-% aus Wasser besteht und wobei
(g) die Temperatur des zweiten Teils der ersten Quenchfraktion geringer als die Austrittstemperatur der zweiten Quenchfraktion ist.

2. Verfahren nach Anspruch 1, **dadurch gekennzeichnet, dass** die Eintrittstemperatur des ersten Quenchmediums in die erste Quenchstufe höher ist als die Eintrittstemperatur des zweiten Quenchmediums in die zweite Quenchstufe.

3. Verfahren nach einem der vorhergehenden Ansprüche, **dadurch gekennzeichnet, dass** der erste Teil der ersten Quenchfraktion vor der Rückführung in die erste Quenchstufe auf eine Temperatur zwischen 20 und 70 °C abgekühlt wird und/oder dass der zweite Teil der ersten Quenchfraktion vor Eintritt in die zweite Quenchstufe auf eine Temperatur zwischen 30 und 50 °C gekühlt wird.

4. Verfahren nach einem der vorhergehenden Ansprüche, **dadurch gekennzeichnet, dass** ein erster Teil der wässrigen Fraktion als zweites Quenchmedium zurück in die zweite Quenchstufe geführt wird und ein zweiter Teil der wässrigen Fraktion einer weiteren Aufreinigung zugeführt wird.

5. Verfahren nach Anspruch 4, **dadurch gekennzeichnet, dass** die weitere Aufreinigung eine Kolonne zur Rückgewinnung von Oxygenaten und/oder eine Wasserreinigung ist.

6. Verfahren nach einem der vorhergehenden Ansprüche, **dadurch gekennzeichnet, dass** der fluide Produktstrom nach dem Austritt aus dem Synthesereaktor und vor Eintritt in die erste Quenchstufe des mehrstufigen Quenchsystems abgekühlt wird.

7. Verfahren nach einem der vorhergehenden Ansprüche, **dadurch gekennzeichnet, dass** der dritte Abflussstrom, der mindestens einen Teil der im Quenchsystem kondensierten Kohlenwasserstoffe enthält, vollständig aus dem Quenchsystem abgeführt wird.

8. Verfahren nach einem der vorhergehenden Ansprüche, **dadurch gekennzeichnet, dass** die Rückführung des ersten Teils der ersten Quenchfraktion in die ersten Quenchstufe mittels Durchflussregelung erfolgt und/oder dass die Rückführung des ersten Teils der wässrigen Fraktion als zweites Quenchmedium in die zweite Quenchstufe mittels Niveauregelung erfolgt.

9. Verfahren nach einem der vorhergehenden Ansprüche, **dadurch gekennzeichnet, dass** die erste Quenchfraktion nach dem Ausleiten aus der ersten Quenchstufe abgekühlt und in einen zweiten Phasenabscheider geleitet wird, wobei eine wasserreiche Flüssigphase erhalten wird, die als erster Teil der ersten Quenchfraktion zu der ersten Quenchstufe zurückgeführt wird und wobei eine kohlenwasserstoffreiche Flüssigphase erhalten wird, die als zweiter Teil der ersten Quenchfraktion mit der zweiten Quenchfraktion aus der zweiten Quenchstufe zu dem Gesamtstrom kombiniert wird.

10. Verfahren nach einem der vorhergehenden Ansprüche, **dadurch gekennzeichnet, dass** der zweite Abflussstrom einer bevorzugt mehrstufigen Kohlenwasserstofffraktionierungsvorrichtung zugeführt und in dieser in olefinhaltige Kohlenwasserstofffraktionen aufgetrennt wird.

## Claims

1. Method for quenching a fluid product stream containing water and hydrocarbons from an oxygenate-to-olefin (OTO) synthesis reactor in a quench system comprising several quench stages, comprising the following steps:
(a) introducing the product stream into a first quench stage, contacting the product stream in the first quench stage with a water-containing first quench medium to form a first quench fraction and a first exit stream, discharging the first quench fraction and the first exit stream from the first quench stage, recycling a first portion of the first quench fraction to the first quench stage as the first quench medium,
(b) introducing the first exit stream into a second quench stage, contacting the first exit stream in the second quench stage with a water-containing second quench medium to form a second quench fraction and a second exit stream, discharging the second quench fraction and the second exit stream from the second quench stage, wherein
(c) a second portion of the first quench fraction not returned to the first quench stage is combined with at least a portion of the second quench fraction from the second quench stage to form an overall stream, and
(d) this overall stream is supplied to at least one first phase separator in which the overall stream is separated into a third liquid exit stream comprising predominantly hydrocarbons and an aqueous fraction, and
(e) at least a first portion of the aqueous fraction is returned as a second quench medium after step (b), wherein
(f) the inlet temperature of the first quench medium into the first quench stage is adjusted such that the first quench fraction consists of water to an extent of at least 70% by weight, preferably at least 80% by weight, most preferably at least 90% by weight, and wherein
(g) the temperature of the second portion of the first quench fraction is lower than the exit temperature of the second quench fraction.

2. Method according to Claim 1, **characterized in that** the inlet temperature of the first quench medium into the first quench stage is higher than the inlet temperature of the second quench medium into the second quench stage.

3. Method according to either of the preceding claims, **characterized in that** the first portion of the first quench fraction, before being recycled to the first quench stage, is cooled to a temperature between 20 and 70°C and/or **in that** the second portion of the first quench fraction, before entering the second quench stage, is cooled to a temperature between 30 and 50°C.

4. Method according to any of the preceding claims, **characterized in that** a first portion of the aqueous fraction is conducted back into the second quench stage as a second quench medium and a second portion of the aqueous fraction is sent to further purification.

5. Method according to Claim 4, **characterized in that** the further purification is a column for the recovery of oxygenates and/or a water purification.

6. Method according to any of the preceding claims, **characterized in that** the fluid product stream is cooled after exiting from the synthesis reactor and before entering the first quench stage of the multistage quench system.

7. Method according to any of the preceding claims, **characterized in that** the third exit stream containing at least a portion of the hydrocarbons condensed in the quench system is fully discharged from the quench system.

8. Method according to any of the preceding claims, **characterized in that** the recycling of the first portion of the first quench fraction to the first quench stage is effected by means of flow control and/or **in that** the recycling of the first portion of the aqueous fraction as a second quench medium to the second quench stage is effected by means of level control.

9. Method according to any of the preceding claims, **characterized in that** the first quench fraction, after being discharged from the first quench stage, is cooled down and directed into a second phase separator to obtain a water-rich liquid phase which is returned to the first quench stage as the first portion of the first quench fraction, and to obtain a hydrocarbon-rich liquid phase which is combined as the second portion of the first quench fraction with the second quench fraction from the second quench stage to give the overall stream.

10. Method according to any of the preceding claims, **characterized in that** the second exit stream is supplied to a preferably multistage hydrocarbon fractionation apparatus and is separated therein into olefinic hydrocarbon fractions.

## Revendications

1. Procédé de refroidissement rapide d'un flux fluide de produits, contenant de l'eau et des hydrocarbures, provenant d'un réacteur de synthèse de type produit oxygéné-à-oléfine (oxygenate-to-olefin - OTO), dans un système de refroidissement rapide comprenant plusieurs étages de refroidissement rapide, comprenant les étapes suivantes :
(a) introduction du flux de produits dans un premier étage de refroidissement rapide, mise en contact du flux de produits dans le premier étage de refroidissement rapide avec un premier agent de refroidissement rapide contenant de l'eau pour la formation d'une première fraction de refroidissement rapide et d'un premier effluent, évacuation de la première fraction de refroidissement rapide et du premier effluent à partir du premier étage de refroidissement rapide, recyclage d'une première partie de la première fraction de refroidissement rapide vers le premier étage de refroidissement rapide en tant que premier agent de refroidissement rapide,
(b) introduction du premier effluent dans un deuxième étage de refroidissement rapide, mise en contact du premier effluent dans le deuxième étage de refroidissement rapide avec un deuxième agent de refroidissement rapide contenant de l'eau pour la formation d'une deuxième fraction de refroidissement rapide et d'un deuxième effluent, évacuation de la deuxième fraction de refroidissement rapide et du deuxième effluent à partir du deuxième étage de refroidissement rapide,
(c) une deuxième partie de la première fraction de refroidissement rapide, non ramenée au premier étage de refroidissement rapide, étant combinée avec au moins une partie de la deuxième fraction de refroidissement rapide provenant du deuxième étage de refroidissement rapide en un flux total et
(d) ce flux total étant amené à un premier séparateur de phases dans lequel le flux total est séparé en un troisième effluent liquide comprenant principalement des hydrocarbures et en une fraction aqueuse et
(e) au moins une première partie de la fraction aqueuse étant ramenée à l'étape (b) en tant que deuxième agent de refroidissement rapide,
(f) la température d'entrée du premier agent de refroidissement rapide dans le premier étage de refroidissement rapide étant réglée de telle sorte que la première fraction de refroidissement rapide est constituée, à raison d'au moins 70% en poids, de préférence à raison d'au moins 80% en poids, le plus préférablement à raison d'au moins 90% en poids, d'eau et
(g) la température de la deuxième partie de la première fraction de refroidissement rapide étant inférieure à la température de sortie de la deuxième fraction de refroidissement rapide.

2. Procédé selon la revendication 1, **caractérisé en ce que** la température d'entrée du premier agent de refroidissement rapide dans le premier étage de refroidissement rapide est supérieure à la température d'entrée du deuxième agent refroidissement rapide dans le deuxième étage de refroidissement rapide.

3. Procédé selon l'une des revendications précédentes, **caractérisé en ce que** la première partie de la première fraction de refroidissement rapide est refroidie avant le recyclage dans le premier étage de refroidissement rapide à une température entre 20 et 70°C et/ou **en ce que** la deuxième partie de la première fraction de refroidissement rapide est refroidie, avant l'entrée dans le deuxième étage de refroidissement rapide, à une température entre 30 et 50°C.

4. Procédé selon l'une des revendications précédentes, **caractérisé en ce qu'**une première partie de la fraction aqueuse est ramenée, en tant que deuxième agent de refroidissement rapide, au deuxième étage de refroidissement rapide et une deuxième partie de la fraction aqueuse est amenée à une purification complémentaire.

5. Procédé selon la revendication 4, **caractérisé en ce que** la purification complémentaire est une colonne pour la récupération de produits oxygénés et/ou une purification à l'eau.

6. Procédé selon l'une des revendications précédentes, **caractérisé en ce que** le flux fluide de produits est refroidi en aval de la sortie du réacteur de synthèse et en amont de l'entrée dans le premier étage de refroidissement rapide du système de refroidissement rapide à plusieurs étages.

7. Procédé selon l'une des revendications précédentes, **caractérisé en ce que** le troisième effluent, qui contient au moins une partie des hydrocarbures condensés dans le système de refroidissement rapide, est complètement évacué à partir du système de refroidissement rapide.

8. Procédé selon l'une des revendications précédentes, **caractérisé en ce que** le recyclage de la première partie de la première fraction de refroidissement rapide dans le premier étage de refroidissement rapide est effectué au moyen d'une régulation de débit et/ou **en ce que** le recyclage de la première partie de la fraction aqueuse en tant que deuxième agent de refroidissement rapide dans le deuxième étage de refroidissement rapide est effectué au moyen d'une régulation de niveau.

9. Procédé selon l'une des revendications précédentes, **caractérisé en ce que** la première fraction de refroidissement rapide, après l'évacuation à partir du premier étage de refroidissement rapide, est refroidie et guidée dans un deuxième séparateur de phases, une phase liquide riche en eau étant obtenue, qui est ramenée en tant que première partie de la première fraction de refroidissement rapide au premier étage de refroidissement rapide, et une phase liquide riche en hydrocarbures étant obtenue, qui est combinée, en tant que deuxième partie de la première fraction de refroidissement rapide, avec la deuxième fraction de refroidissement rapide provenant du deuxième étage de refroidissement rapide, en flux total.

10. Procédé selon l'une des revendications précédentes, **caractérisé en ce que** le deuxième effluent est amené à un dispositif, de préférence à plusieurs étages, de fractionnement d'hydrocarbures et est séparé dans celuici en fractions hydrocarbonées contenant des oléfines.
